# EUROPEAN PATENT APPLICATION

(11) **EP 4 721 813 A1**
(43) Date of publication of application: **08.04.2026**
(21) Application number: 24815829.7
(22) Date of filing: 27.05.2024
(51) Int. Cl.: A61N 1/39, A61N 1/362, G16H 20/30, G16H 40/40, G16H 40/67, G06Q 50/10, G06Q 50/22, H04N 7/18

(54) **DEFIBRILLATOR MANAGEMENT SYSTEM USING CAMERA**

(30) Priority: 31.05.2023 KR 20230070230
(71) Applicant: Mediana Co., Ltd., Wonju-si, Gangwon-do 26365 (KR)
(72) Inventor: LEE, Sung Ho, Wonju-si Gangwon-do 26449 (KR); LEE, Hee Tack, Wonju-si Gangwon-do 26389 (KR)
(74) Representative: Gulde & Partner
(86) International application number: PCT/KR2024/007180
(87) International publication number: WO 2024/248453

(57) **Abstract**

The present invention relates to an automated external defibrillator (AED) management system utilizing a camera. This system is designed for spaces such as elevators equipped with both a camera and an AED, where the camera periodically photographs the AED status display unit. This image data is then transmitted to an image data acquisition unit, which subsequently forwards it to an AED management server for status checks and overall management of the AED.

The AED management system according to the present invention comprises an AED box housing, a camera, an image data acquisition unit and AED management unit. The AED box housing that houses the AED, includes an AED status display unit, which comprises an AED fault indicator to display whether the AED is malfunctioning. The camera captures images of the AED box, including the AED status display unit. The image data acquisition unit periodically gathers the images captured by the camera. The AED management unit includes a processing unit that extracts the image of the AED status display unit from the camera images received from the image data acquisition unit and determines the AED operational status using the image of the AED fault indicator contained within the AED status display unit image.

A key advantage of the present invention is its ability to manage AEDs even in environments where the installation of additional network devices is restricted due to regulatory limitations and safety concerns, such as in elevators. Furthermore, this system enables periodic AED management without requiring new AEDs, allowing for the continued use of existing units.

## Description

### [Technical Field]

<1> The present invention relates to a defibrillator management system utilizing a camera. This system periodically photographs the status display unit of a defibrillator using a camera installed in a space, such as an elevator, where both a camera and a defibrillator are present. These images are transmitted to an image data acquisition unit, which then forwards them to a defibrillator management server. Using the transmitted image data, the defibrillator management server checks (inspection) and manages the status of the defibrillator.

### [Background Art]

<2> Cardiac arrest leads to an immediate cessation of blood circulation throughout the body, which, without prompt intervention, can result in death or severe brain damage. The brain, in particular, is highly susceptible to irreversible damage if blood supply is interrupted for as little as 4-5 minutes.

<3> An Automated External Defibrillator (AED) is a device that typically provides voice prompts to guide users through CPR. It is designed to check the patient's heart condition and automatically deliver an electric shock if needed.

<4> Generally, for every minute that passes after cardiac arrest, the survival rate decreases by approximately 10%. Therefore, it is crucial to administer CPR and defibrillation using an AED within five minutes. Consequently, accessibility to AEDs is paramount for improving survival rates. Recently, AEDs have been installed in various indoor locations such as elevators, restaurants, and offices, often accompanied by surveillance cameras.

<5> Notably, the home is the most common location for cardiac arrest, accounting for approximately 60% of all incidents. As a result, AEDs are frequently placed in elevators in public housing complexes.

<6> However, since cardiac arrest incidents are not frequent, there is a risk that an AED may malfunction or fail to operate due to a depleted battery or other issues when an actual emergency occurs. This necessitates periodic status checks and maintenance of AEDs.

<7> Most AEDs have an AED status display unit, a small screen, either on the AED itself or on its storage box (AED box housing). The AED status display unit shows important information like if the AED is working correctly or how much battery it has left. Usually, someone in charge of the AED has to go to where it's kept and look at this screen to check on it. But it's hard to check them often because someone has to physically go to each AED or its storage box. Because of this, people are now looking into ways to manage AEDs using the internet or wireless communication.

<8> Nevertheless, installing additional network equipment in elevators poses difficulties due to regulatory restrictions and safety management concerns, making AED management challenging in these environments.

<9> Therefore, there is a recognized need for a method to manage AEDs by utilizing already installed cameras (CCTV systems).

<10> The present invention proposes an AED management system that leverages existing cameras. In spaces such as elevators where both a camera and an AED are installed, the camera periodically captures images of the AED's status display. These image data are then transmitted to an image data acquisition unit, which in turn forwards them to an AED management server for status verification and management.

<11> Korean Registered Patent No. 10-1155022 is about an operation system for defibrillator. This system connects to and manages a defibrillator storage box. Because of this, Korean Registered Patent No. 10-1155022 is hard to use in places where there is no internet or wireless communication.

### [Disclosure]

### [Technical Problem]

<12> The agenda of the present invention aims to provide a defibrillator management system using a camera, wherein the camera periodically photographs a status display unit of a defibrillator in a space where the camera and the defibrillator are installed, transmits the photographed image (camera image) data to an image data acquisition unit, and transmits the image data from the image data acquisition unit to a defibrillator management server (AED management unit) to perform status inspection (check) and management of the defibrillator.

### [Solution to Problem]

<13> To solve the problem mentioned above, the AED management system using a camera, as described in this invention, includes: an AED box housing that holds an AED (Automated External Defibrillator) and has an AED status display that shows if the AED is broken; a camera that takes pictures of the AED box housing, including its AED status display unit; an image data acquisition unit that regularly gathers the images (videos) taken by the camera; and an AED management unit that includes a processing unit that takes the AED status display unit image from the camera image it gets from the image data acquisition unit. The processing unit determines whether the AED is faulty by using the image of the AED fault indicator included in the image of the AED status display unit.

<14> The AED status display unit also has a battery level indicator that shows how much battery charge is left. The processing unit uses this picture (camera image) of the battery level indicator to figure out how much power the AED battery has.

<15> The AED status display unit also has a pad fault indicator that shows if the pads are working correctly. The processing unit uses this picture of the pad fault indicator to check if the electrode pads are faulty.

<16> The AED box housing has a transparent window on its upper cover. This window acts as a display to show if an AED is inside the case. The processing unit gets camera images and looks at the window's image. By looking at this image, processing unit can tell if an AED is stored inside its case.

<17> The AED management unit transmits an alarm signal to the administrator's personal terminal if an AED is determined to be faulty, if its battery level is depleted, if the electrode pads are deemed defective, or if the AED is not housed within its box.

<18> The processing unit can apply the image from the AED status display to a pre-trained fault discrimination artificial intelligence model, with the model's output serving as the fault determination result. Alternatively, the processing unit can perform pattern recognition on the image from the AED fault status indicator by comparing it with a previously stored image of the AED fault status indicator of a normally functioning AED. If the image from the AED fault status display differs from the image of the AED fault status indicator of a normally functioning AED, the AED can be determined to be faulty.

<19> The camera is a CCTV.

<20> The present invention features a method for operating a defibrillator management system for administering an Automated External Defibrillator (AED), which the system includes an AED box housing the AED and equipped with an AED status display unit. The method for operating the defibrillator management system comprising: AED status display unit image extraction step, wherein the processing unit extracts the image of the AED status display unit from the camera image captured by the camera; AED status display unit image analysis step, wherein the processing unit extracts an image of the AED fault indicator from the AED status display unit image extracted in the AED status display unit image extraction step, and determines whether the AED is normal or has a fault from the extracted image of the AED fault indicator; and AED fault notification step, wherein if the AED is judged to be faulty in the AED status display unit image analysis step, the processing unit transmits an alarm notifying that the AED is faulty to an administrator personal terminal (200).

<21> The AED status display unit image analysis step includes the processing unit extracting an image of the battery level indicator from the AED status display unit image, determining the remaining battery level from the extracted battery level indicator image, and determining if the remaining battery level is below a preset threshold, and if the remaining battery level is below the threshold, determining that it is time for battery replacement. The AED fault notification step includes that the processing unit transmits an alarm notifying that it is time to replace to the administrator's personal terminal (200) the battery if the processing unit determined that it is time to replace the battery in the AED status display unit image analysis step.

<22> The AED status display unit image analysis step involves the processing unit extracting an image of the pad defect indicator from the AED status display unit image and determining a pad defect if a defect indication is detected within the extracted image of the pad defect indicator. The AED fault notification step further comprises the processing unit transmitting an alarm indicating a pad defect to the administrator's personal terminal (200) if a pad defect is determined in the AED status display unit image analysis step.

<23> The AED status display unit image analysis step can be characterized in that the processing unit applies the image of the AED status display to a pre-trained fault discrimination artificial intelligence model and uses the output of the fault discrimination artificial intelligence model as the fault determination result. Also, in another way, the AED status display unit image analysis step can be characterized in that the processing unit performs pattern recognition on the image of the AED fault indicator against a pre-stored normal image of the AED fault indicator image, and if the image of the AED fault indicator differs from the pre-stored normal image, the AED is determined to be faulty.

<24> The AED box housing features a transparent window on its upper cover, namely, AED presence indicator, allowing the user to ascertain whether an AED is housed within the box housing. The AED status display unit image analysis step comprises the processing unit extracting an image of the transparent window of the AED box housing from the camera image received from the image data acquisition unit, and using the extracted transparent window image to determine whether the AED is housed within the AED box housing. The AED fault notification step comprises the processing unit transmitting an alarm to the administrator's personal terminal (200) indicating that the AED is not housed within the AED box housing, if it is determined in the AED status display unit image analysis step that the AED is not housed within the AED box housing.

### [Advantageous Effects]

<25> In the present invention, the defibrillator management system utilizing a camera periodically photographs the status display unit of the defibrillator by the camera and transmits the image data to an image data acquisition unit and transmits the image data from the image data acquisition unit to a defibrillator management to perform status inspection (check) and management of the defibrillator.

<26> Consequently, this invention enables the management of AEDs even in scenarios where the installation of additional network devices is restricted, such as in elevators, due to regulatory constraints and safety management concerns.

<27> Furthermore, a significant advantage of this invention is that it facilitates periodic management without requiring the acquisition of new AEDs, as it can be seamlessly integrated with existing AED units.

### [Brief Description of the Figures]

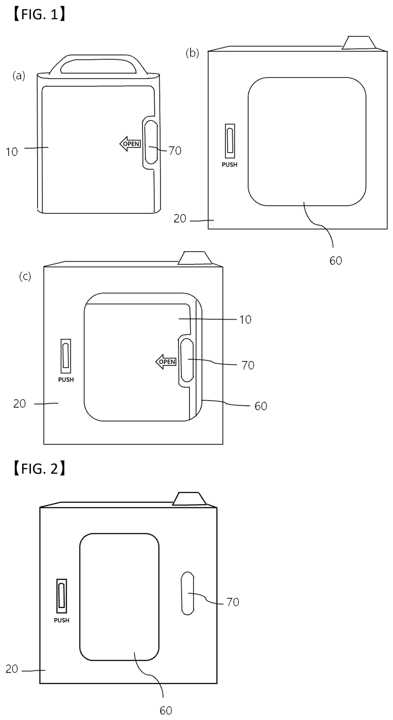

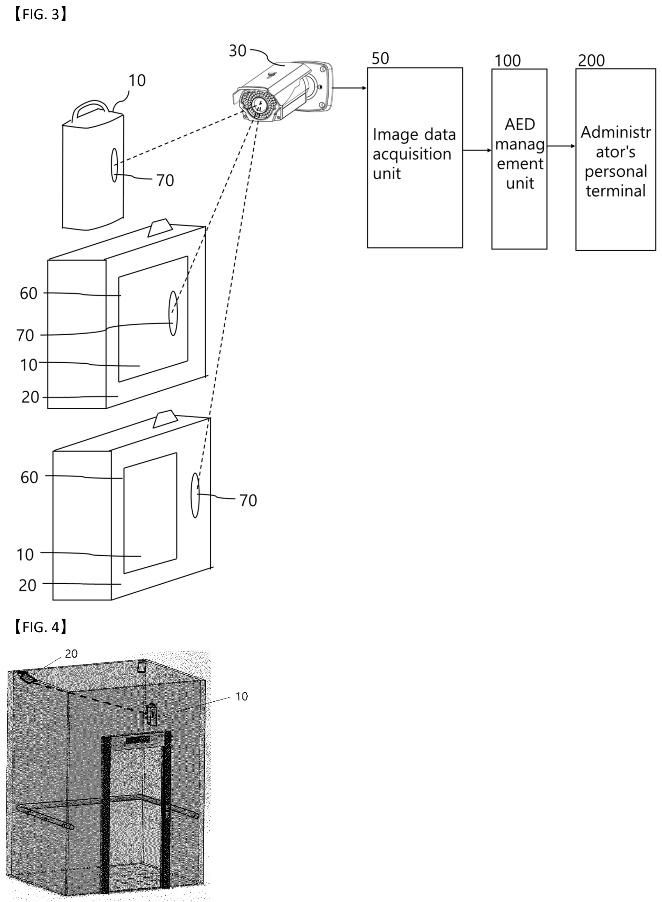

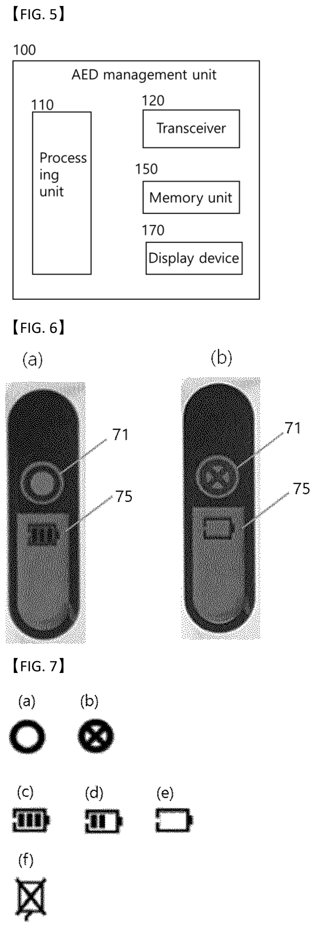

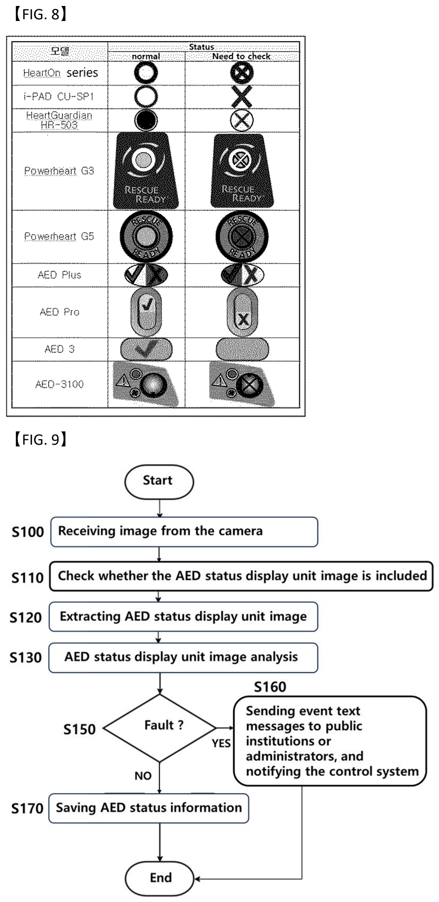

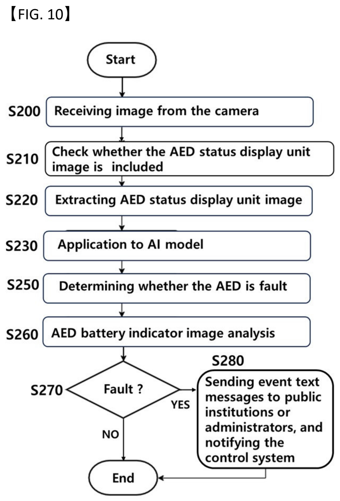

<28> Figure 1 is an explanatory diagram illustrating an AED status display unit of an AED visible through a transparent window of an AED box housing, according to one embodiment of the AED box.
<29> Figure 2 is an explanatory diagram illustrating an AED status display unit equipped in an AED box housing, according to another embodiment of the AED box housing.
<30> Figure 3 is a schematic explanatory diagram illustrating an AED management system utilizing a camera according to the present invention.
<31> Figure 4 is a diagram illustrating an exemplary operational state of an AED management system utilizing a camera according to the present invention.
<32> Figure 5 is a block diagram schematically illustrating the configuration of the AED management unit (100) shown in figure 3.
<33> Figure 6 shows examples of images from the AED status display unit (70).
<34> Figure 7 shows examples of indicators displayed on the AED status display unit (70).
<35> Figure 8 shows examples of images from the AED fault indicator of a commercially available AED.
<36> Figure 9 is an exemplary flowchart illustrating how the processing unit in figure 5 determines the fault status of an AED.
<37> Figure 10 is another exemplary flowchart illustrating how the processing unit in figure 5 determines the fault status of an AED.

### [Specific Details for Implementation of the Invention]

<38> Hereinafter, the defibrillator management system utilizing a camera according to the present invention will be described in detail with reference to the accompanying figures.

<39> Figure 1 is an explanatory diagram illustrating an AED status display unit of an AED visible through the transparent window of an AED box housing, representing one embodiment of an AED box housing. Figure 2 is an explanatory diagram illustrating an AED status display unit provided in an AED box housing, representing another embodiment of an AED box housing. Figure 3 is a schematic explanatory diagram illustrating the defibrillator management system utilizing a camera according to the present invention.

<40> Figure 1(a) shows an AED (10), Figure 1(b) shows an example of an AED box housing (20), and Figure 1(c) shows an example of the AED (10) inserted into the AED box housing (20) of Figure 1(b).

<41> The AED (10) is a device capable of performing cardiopulmonary resuscitation and is configured to automatically deliver an electrical shock after checking the patient's heart condition. Generally, it includes an AED status display unit (70) that displays the remaining battery life and whether the AED is malfunctioning.

<42> The AED (10) assesses the remaining battery life of the AED and any malfunctions, displaying this information on the AED status display unit (70).

<43> The AED box housing (20) is a container designed to house and store an AED internally and typically features a transparent window (60) on its front.

<44> As shown in Figure 1, the AED box housing (20) allows for verification of the AED (10) contained within through the transparent window (60). Specifically, the AED status display unit (70) of the AED (10) can be viewed through the transparent window (60).

<45> In some cases, the AED box housing (20) may be equipped with an AED status display unit (70) on the AED box housing (20), as shown in FIG. 2. In such instances, the AED (10) and AED box housing (20) are provided, allowing the AED box housing (20) to check the status of the AED (10) and output it to the AED status display unit (70) located on one outer side of the AED box housing (20).

<46> Generally, when an AED (10) is installed indoors, it can either be placed standalone or housed within an AED box housing (20).

<47> The camera (30) is positioned to capture the AED status display unit (70) visible through the transparent window (60) of the AED box housing (20), or the AED status display unit (70) of the AED (10), or the AED status display unit (70) of the AED box housing (20). The captured camera images are periodically transmitted to the image data acquisition unit (50). The camera's image includes the AED status display unit (70). The camera (30) is a Closed-circuit Television (CCTV) system, and can utilize a webcam, a general-purpose camera, or similar devices.

<48> The camera (30), i.e., CCTV, captures video (moving images) at predetermined intervals to determine whether the AED is functioning normally, including the AED status display unit (70). If this interval matches the flashing time of the camera's (30) LED, the video can be divided into 10-second segments for capture to assess the AED's operational status, whether it is normal or not.

<49> The image data acquisition unit (50) is a means for receiving video data from the camera (30). It receives the video of the AED status display unit from the camera (30) and transmits it to the AED management unit (100).

<50> In some instances, the image data acquisition unit (50) may be integrated into an elevator management system or a building management system.

<51> The AED management unit (100) extracts images of the AED status display unit (70) from the camera image received from the image data acquisition unit (50). From these, it further isolates images of the AED fault indicator (71) and the battery level indicator (75) to determine the AED's operational status (failure status), current battery level, and whether battery replacement is due. The results are then stored in the memory unit (150) for each individual AED and can be transmitted to the display device (170) or to the administrator's personal terminal (200). The administrator's personal terminal (200) may be a smartphone or a personal computer.

<52> Figure 4 illustrates an example of the operational state of a defibrillator management system utilizing the camera of the present invention.

<53> In Figure 4, a camera (30) installed in an elevator captures the AED status display unit (70) through the transparent window (60) of the AED box housing (20) and transmits the video data to the image data acquisition unit (50).

<54> Figure 5 is a block diagram schematically illustrating the configuration of the AED management unit (100) shown in Figure 3.

<55> The processing unit (execution processing unit, or operation processing unit) (110) receives camera image via the transceiver (120) from the image data acquisition unit (50). It then preprocesses the camera video to extract only the region of interest, determines whether the image within this region of interest includes the AED status display unit (70), and subsequently extracts the image of the AED status display unit.

<56> The processing unit (110) makes an analysis from the image of the AED status display unit (70). From this image, it takes out the image of the AED fault indicator (71) and the image of the battery level indicator (75). The processing unit (110) then figures out if the image from the AED fault indicator (71) shows a fault (error, malfunction) status, or if it shows a normal (working fine) status. Here, the fault status is that the AED is not working correctly, and the normal status is that the AED is working correctly and there are no errors. If the image of the AED fault indicator is decided the image indicating a fault, the processing unit decides the AED to a fault status. If the image of the AED fault indicator is decided the image indicating a normal, the processing unit decides the AED to a normal status. Then, the decided result is saved for each AED in the memory unit (150) and transmitted to the administrator's personal terminal (200) or other places through the transceiver (120). Sometimes, the processing unit (110) might use artificial intelligence (like an Al model that decides defects) to decide if the AED is malfunctioning or normal, based on the image of the AED fault indicator (71).

<57> The processing unit (110) determines the remaining battery level from the image of the battery level indicator (75). For example, the battery level indicator (75) indicates the remaining battery level by drawing a line within the outer line (border) of the battery shape. If the remaining battery level on the image of the battery level indicator (75) is below a preset threshold, the processing unit (110) determines that it is time to replace the battery, stores the remaining battery level and whether it is time to replace the battery in the memory unit (150) for each AED, and transmits it to the administrator personal terminal (200) or the like through the transceiver unit (120). In some cases, the processing unit (110) may apply artificial intelligence (i.e., an artificial intelligence model for determining the remaining battery level) to determine the remaining battery level of the battery level indicator (75).

<58> Figure 6 illustrates examples of the display screens on the AED status display unit (70).

<59> Figure 6(a) shows the AED status display unit (70) that the AED fault indicator displays a normal operating status image (i.e., indicating the image that the AED is functioning correctly) and the battery level indicator displays a full battery level image, denoted as "FULL" status image. Conversely, Figure 6(b) depicts the AED status display unit (70) displaying a fault status (i.e., indicating an AED malfunction) alongside an empty battery level.

<60> Normally, an AED shows it's working fine with a blue (or green) circle "∘" or a checkmark "√". If there's a problem, namely in a fault status, it will show a red (or black) "×" or a circled " ⓧ".

<61> Sometimes, the AED status display unit (70) might also have a pad fault indicator (not shown) that indicates whether the electrode pad is normal or defective.

<62> The processing unit (110) checks the image of the pad fault indicator (not shown) and, if it detects a display indicating a pad defect, transmits an alarm to the administrator personal terminal (200) or the like to notify this.

<63> Also, the AED box housing (20) has a transparent window (60) that shows if the AED is not inside it. This transparent window (60) is on the upper cover of the AED box housing (20), so you can easily see if the AED is in the box.

<64> If the AED is not seen through the transparent window (60) - meaning someone has taken the AED out of the box (20) and moved it - the processing unit (110) will immediately send an alarm to the administrator's personal terminal (200) and other places to let them know.

<65> Figure 7 shows examples of indicators displayed on the AED status display unit (70).

<66> Figure 7 (a) is an example of a display indicating that the AED is normal (working fine), and Figure 7 (b) is an example of a display indicating that the AED is malfunctioning.

<67> Figure 7 (c) shows that the AED battery level is "FULL" status, Figure 6 (d) shows that the battery level has partially depleted, and Figure 7 (e) shows an example of a battery level with no remaining charge.

**<68>** **Figure** 7 (f) shows an example of a defective electrode pad.

<69> Figure 8 presents examples of AED fault indicator displays found on AEDs commercially available from various manufacturers.

<70> As shown in Figure 8, methods for indicating a normal AED status on the AED fault indicator (71) vary, including ring-shaped, circular, a combination of ring and circle, and checkmark (√, tick) shapes.

<71> Furthermore, methods for indicating that an AED requires inspection-that is, a malfunction (fault)-on the AED fault indicator (71) include an "X" shape, an "X" within a circle (i.e., ⓧ), and an "X" within a ring.

<72> Generally, the AED performs a self-diagnosis, and the indicators on the AED status display unit (70) may change.

<73> The processing unit (110) extracts the image of the AED fault indicator (71) from the image of the AED status display unit (70) and determines whether the image of the AED fault indicator (71) displays a preset normal indication image or a normal indication color. For example, the image of the AED fault indicator (71) is input into a pre-trained fault discrimination artificial intelligence model, and the output from the fault discrimination artificial intelligence model is provided as a fault detection value (a value indicating whether a fault or normal operation is present).

<74> The fault discrimination artificial intelligence model is trained using data consisting of pairs of images of the AED fault indicator (71) of various commercially available AEDs and corresponding fault detection values.

<75> Furthermore, the processing unit (110) extracts the image of the battery level indicator (75) from the image of the AED status display unit (70) and determines the remaining charge level from the image of the battery level indicator (75). For instance, the image of the battery level indicator (75) is input into a pre-trained battery level detection artificial intelligence model, and the output from the battery level detection artificial intelligence model is provided as a battery level value.

<76> The Al model for determining battery levels is trained using a dataset consisting of pairs of images from the battery level indicator (75) of various commercially available AEDs and their corresponding battery level values.

<77> Figure 9 illustrates an exemplary flowchart for the processing unit in Figure 5 to determine AED malfunction.

<78> In the step for determining the inclusion of an AED status display unit image, the processing unit (110) receives camera image from the image data acquisition unit (50), preprocesses it to extract only the region of interest (S100), verifies if the image of the region of interest includes the image of the AED status display unit (70) (S110), and then extracts the image of the AED status display (S120).

<79> In the AED status display unit image analysis step, the processing unit (110) extracts an image of the AED fault indicator (71) from the AED status display image identified in the step of determining the inclusion of an AED status display unit image. From this extracted image of the AED fault indicator (71), it determines whether the AED is functioning normally or if there is a fault. Additionally, from the AED status display image, the processing unit extracts an image of the battery level indicator (75). It then determines the remaining battery level from the extracted image of the battery level indicator (75) and assesses whether this level is below a pre-set threshold. If it is, the processing unit determines that it is time to replace the battery(S130). In other words, the system determines that the battery requires replacement. Furthermore, in this AED status display unit image analysis step, the processing unit (110) checks the image of the pad defect indicator (not shown) and, upon detecting an indication of a pad defect, determines that a pad defect is present.

<80> In some instances, to determine whether an AED is functioning correctly or is faulty, the processing unit performs pattern recognition by comparing the extracted image of the AED fault indicator (71) with a previously stored image of the AED fault indicator (71) of a normally operating AED. If the patterns match, the AED is determined normal and if they differ, it is determined faulty. Alternatively, the processing unit performs pattern recognition by comparing the extracted image of the AED fault indicator (71) with a previously stored image of the AED fault indicator (71) of a faulty operating AED. If the patterns match, the AED is determined faulty and if they differ, it is determined normal. In this way, the processing unit can also use pattern recognition to determine whether in the pad defect indicator image, the pad is defective.

<81> In the AED normal status determination step, the processing unit (110) assesses whether the AED has been determined to be faulty in the AED status display unit image analysis step, and also determines if it is time for a battery replacement. If the AED is determined to be faulty, or if it is time for a battery replacement, or if the electrode pads are deemed defective, the process proceeds to the AED fault notification step (S150).

<82> In the AED status information storage step, if the processing unit (110) determines in the AED normal status determination step that the AED is not faulty, it is not time for a battery replacement, and the electrode pads are not defective, then a value indicating the AED's normal status and the remaining battery level detected in the AED status display unit image analysis step are stored as AED status information in the memory unit (150) (S170), and the process concludes.

<83> In the AED fault notification step, the processing unit (110) transmits AED status information indicating the AED's fault condition, or information indicating it is time for battery replacement, or an event message stating that AED status inspection is required, to the administrator's personal terminal (200), or to a public institution terminal (not shown) connected to the defibrillator management system, or to other control systems (not shown) connected to the defibrillator management system (S160), and the process concludes.

<84> Other control systems may include building management systems, apartment management systems, elevator management systems, and so forth.

<85> Figure 10 illustrates another example of a flowchart detailing how the processing unit of Figure 5 determines the operational status of an AED.

<86> In the step for determining the inclusion of an AED status display unit image (namely, AED status display unit image extraction step), the processing unit (110) receives camera image from the image data acquisition unit (50), preprocesses it, and extracts only the region of interest (S200). It then verifies whether the image of the AED status display unit (70) is present within the extracted region of interest (S210) and subsequently extracts the AED status display image from the region of interest (S220).

<87> In some instances, the process of verifying the inclusion of the AED status display unit (70) image within the region of interest (S210) can be performed using artificial intelligence (e.g., an Al model designed for AED status display detection).

<88> During the AED fault indicator image determination step (namely, AED status display unit image analysis step), the processing unit (110) applies the AED status display unit image, extracted in the step for determining the inclusion of an AED status display unit image to a pre-trained fault detection Al model (S230). The output of this fault detection Al model is then used as the fault determination result to assess whether the AED is malfunctioning (S250).

<89> In the AED fault indicator image determination step, the processing unit (110) applies the AED status display unit image, extracted during the AED status display unit image inclusion determination step, to a pre-trained fault detection Al model (S230). The output of this fault detection AI model serves as the result for determining the AED's operational status, which is then stored in the memory unit (S250).

<90> In the AED battery level indicator image determination step, the processing unit (110) apply the AED status display image, extracted In the step for determining the inclusion of an AED status display unit image, to a pre-trained battery level discrimination artificial intelligence model. The output of the fault discrimination artificial intelligence model is designated as the battery level. The unit then determines if the battery level is below a preset threshold, and if it is, the system identifies this as the time for battery replacement and stores the battery level and replacement timing information in the memory unit (S260).

<91> In the AED normal status determination step, the processing unit (110) assesses whether the AED was determined to be faulty in the AED fault indicator image determination step, and also whether it is time to replace the battery in the AED battery level indicator image determination step. If the AED is determined not to be faulty and battery replacement is not required, the process concludes (S270).

<92> In the AED fault notification step, the processing unit (110) transmits AED status information indicating a fault, or information indicating that battery replacement is due, or an event message stating that an AED status check is required, to the administrator's personal terminal (200), a public institution terminal (not shown) connected to the defibrillator management system, or other control systems (not shown) connected to the defibrillator management system (S280), and then terminates the process.

<93> The present invention has been illustrated and described above with reference to specific preferred embodiments. However, the present invention is not limited to the mentioned embodiments, and it will be apparent to those skilled in the art that various modifications can be made without departing from the spirit and scope of the invention as defined by the appended claims.

### [Industrial Utility]

<94> The defibrillator management system utilizing a camera, as described herein, is designed to periodically inspect and manage the status of defibrillators installed in designated areas, such as elevators, using a camera. This system is broadly applicable and can be implemented in various locations, including elevators, schools, hospitals, companies, and shopping centers. Notably, if a camera is already present in a given space, this invention eliminates the need for a new automated external defibrillator (AED) for management purposes, allowing for the periodic management of existing AEDs.

## Claims

1. An defibrillator management system utilizing a camera, comprising:
an AED status display unit, wherein is equipped in an automated external defibrillator (AED) or an AED box housing and storing the AED, and includes an AED fault indicator for displaying whether the AED is malfunctioning;
a camera for capturing images, including those of the AED status display unit;
an image data acquisition unit for periodically collecting images captured by the camera; and
an AED management unit, wherein includes a processing unit for extracting images of the AED status display unit from the camera images received from the image data acquisition unit, and for determining the AED's operational status using the image of the AED fault indicator contained within the image of the AED status display unit.

2. The defibrillator management system according to claim 1,
wherein the AED status display unit includes a battery level indicator, and the processing unit determines the remaining charge of the AED battery using the image of the battery level indicator included in the image of the AED status display unit.

3. The defibrillator management system according to claim 2,
wherein the AED status display unit further comprises a pad fault indicator, and the processing unit determines the fault status of the electrode pads by analyzing the image of the pad fault indicator included in the image of the AED status display.

4. The defibrillator management system according to Claim 3,
wherein the AED box housing has a transparent window on its upper cover, allowing visibility of whether an AED is stored inside the AED box housing.

5. The defibrillator management system according to claim 4,
wherein the processing unit extracts an image of the transparent window of the AED box housing from the camera image received from the image data acquisition unit, and determines whether an AED is housed within the AED box housing using the extracted image of the transparent window.

6. The defibrillator management system according to claim 5,
wherein the AED management unit sends an alarm to the administrator's personal terminal if the AED is determined to be malfunctioning, the AED battery level is determined to be depleted, the electrode pads are determined to be defective, or the AED is determined not to be housed within the AED box housing.

7. The defibrillator management system according to claim 6,
wherein the processing unit applies the image from the AED status display unit to a pre-trained fault discrimination artificial intelligence model and uses the output of the fault detection artificial intelligence model as the fault determination result.

8. The defibrillator management system according to claim 6,
wherein the processing unit performs pattern recognition between the image of the AED fault indicator and a pre-stored AED fault indicator image of a normally operating AED, and determines the AED to be faulty if the image of the AED fault indicator differs from the pre-stored AED fault indicator image.

9. The defibrillator management system according to claim 1,
wherein the camera is a CCTV camera.

10. The defibrillator management system according to claim 4,
wherein the image of the AED status display unit within the image captured by the camera is an image captured from an AED status display unit equipped on the AED or AED box housing, or an image captured through a transparent window of the AED box housing, showing the AED status display unit of the AED within the transparent window.

11. A method for operating a defibrillator management system utilizing a camera that the defibrillator management system includes a processing unit that determines whether an AED (automated external defibrillator) is malfunctioning (fault) by using a captured camera image including an AED status display unit located on the AED or on an AED box housing, the method for operating the defibrillator management system comprising:
AED status display unit image extraction step, wherein the processing unit extracts the image of the AED status display unit from the camera image captured by the camera;
AED status display unit image analysis step, wherein the processing unit extracts an image of the AED fault indicator from the AED status display unit image extracted in the AED status display unit image extraction step, and determines whether the AED is normal or has a fault from the extracted image of the AED fault indicator; and
AED fault notification step, wherein if the AED is judged to be faulty in the AED status display unit image analysis step, the processing unit transmits an alarm notifying that the AED is faulty to an administrator personal terminal (200).

12. The method for operating the defibrillator management system according to claim 11, wherein:
the AED status display unit image analysis step includes the processing unit extracting an image of the battery level indicator from the AED status display unit image, determining the remaining battery level from the extracted battery level indicator image, and determining if the remaining battery level is below a preset threshold, and if the remaining battery level is below the threshold, determining that it is time for battery replacement; and
the AED fault notification step includes that the processing unit transmits an alarm notifying that it is time to replace to the administrator's personal terminal (200) the battery if the processing unit determined that it is time to replace the battery in the AED status display unit image analysis step.

13. A method for operating the defibrillator management system according to claim 12, wherein:
the AED status display unit image analysis step involves the processing unit extracting an image of the pad defect indicator from the AED status display unit image and determining a pad defect if a defect indication is detected within the extracted image of the pad defect indicator; and
the AED fault notification step further comprises the processing unit transmitting an alarm indicating a pad defect to the administrator's personal terminal (200) if a pad defect is determined in the AED status display unit image analysis step.

14. The method for operating the defibrillator management system according to claim 11,
wherein the AED status display unit image analysis step is **characterized in that** the processing unit applies the image of the AED status display to a pre-trained fault discrimination artificial intelligence model, and uses the output of the fault discrimination artificial intelligence model as the fault determination result.

15. The method for operating the defibrillator management system according to claim 11,
wherein the AED status display unit image analysis step involves the processing unit performing pattern recognition on the image of the AED fault indicator against a pre-stored AED fault indicator image of a normal operating AED, and if the image of the AED fault indicator differs from the pre-stored AED fault indicator image, the AED is determined to be faulty.

16. The method for operating the defibrillator management system according to claim 12,
wherein the AED box housing features a transparent window on its upper cover, allowing the user to ascertain whether an AED is housed within the box housing.

17. The method of operating the defibrillator management system according to claim 16, wherein:
the AED status display unit image analysis step comprises the processing unit extracting an image of the transparent window of the AED box housing from the camera image received from the image data acquisition unit, and using the extracted transparent window image to determine whether the AED is housed within the AED box housing; and
the AED fault notification step comprises the processing unit transmitting an alarm to the administrator's personal terminal (200) indicating that the AED is not housed within the AED box housing, if it is determined in the AED status display unit image analysis step that the AED is not housed within the AED box housing.

18. The method for operating the defibrillator management system according to claim 11,
wherein the AED status display unit image extracted in the AED status display unit image extraction step is an image captured of an AED status display unit provided on the AED or AED box housing, or an image captured of the transparent window of the AED box housing, thereby capturing the AED status display unit of the AED located within the transparent window.
